# FASCICULE DE BREVET EUROPEEN

(11) **EP 0 604 631 B1**
(45) Date de publication et mention de la délivrance du brevet: **22.11.2000**
(21) Numéro de dépôt: 93916021.4
(22) Date de dépôt: 20.07.1993
(51) Int. Cl.: A61K 35/74, A61K 39/39, C12P 1/04

(54) **Utilisation d'enveloppes de bactéries de l'ordre des Beggiatoales ou du genre Vitreoscilla comme ingrédient actif d'un médicament destiné à stimuler l'immunité non-spécifique**
Verwendung von Bakterienhüllen der Ordnung Beggiatoales oder der Gattung Vitreoscilla als aktiven Bestandteil eines Medikamentes zur Stimulierung der unspezifischen Immunität
Use of envelopes of bacteria of the order of Beggiatoales or of the genus Vitreoscilla as an active ingredient of a medicament for stimulating non-specific immunity

(30) Priorité: 20.07.1992 FR 9208932
(43) Date de publication de la demande: 06.07.1994
(73) Titulaire: L'OREAL, 75008 Paris (FR)
(72) Inventeur: AUBERT, Lucien, F-06320 Cap-d'Ail (FR); MARTIN, Richard, F-37290 Vouvray (FR)
(74) Mandataire: Tonnellier, Jean-Claude
(86) Numéro de dépôt international: FR9300741
(87) Numéro de publication internationale: WO9402158

(56) Documents cités:
- GB-A- 2 034 687
- BIOLOGICAL ABSTRACTS vol. 85 , 1988, Philadelphia, PA, US; abstract no. 30874, GEORGIOU, C. D. ET AL. 'IDENTIFICATION OF B, C, AND D CYTOCHROMES IN THE MEMBRANE OF VITREOSCILLA'

## Description

L'invention a pour objet l'utilisation d'enveloppes ou de fractions d'enveloppes de bactéries de l'ordre des Beggiatolales ou du genre Vitreoscilla, comme ingrédient actif dans la préparation d'un médicament destiné à stimuler l'immunité non spécifique. L'invention concerne aussi un médicament contenant des enveloppes ou des fractions d'enveloppes de bactéries du genre Vitreoscilla.

On sait que certains constituants des enveloppes des bactéries Gram négatives, et notamment les fractions lipopolysacharidiques qui peuvent en être extraites, présentent des propriétés de stimulation non spécifique des défenses immunitaires de l'organisme ; voir par exemple Int.Archs Allergy Appl. Immun.76 Suppl.1.119-127, (1985), et Journal of Immunopharmacology 3(2), 119-132 (1981).

On a maintenant découvert que les enveloppes de certaines bactéries appelées, selon la classification du Bergey's Manual, bactéries filamenteuses non photosynthétiques et non fructifiantes, présentent des propriétés immunomodulatrices particulièrement intéressantes. Elles stimulent notamment, de façon non spécifique, les défenses immunitaires, y compris l'immunité cellulaire, et en particulier les macrophages.

On appelle ici "enveloppe" la paroi bactérienne et éventuellement les membranes sous-jacentes.

Parmi les bactéries dont les enveloppes ou fractions d'enveloppes sont utilisables conformément à l'invention, on citera les bactéries appartenant à l'ordre des Beggiatoales, et notamment les bactéries appartenant au genre Beggiatoa, telles que par exemple diverses souches de Beggiatoa alba suivant la définition donnée dans Arch. Microbiol. (1984) 137, 139-144. Il convient de noter que cette définition de B.alba correspond aux anciennes appellations Beggiatoa arachnoidea, B. gigantea, B.leptomiformis, B.minima, B.mirabilis du Bergey's manual, 8e édition.

On peut citer par ailleurs les bactéries appartenant au genre Vitreoscilla, dont on sait qu'il est proche et souvent difficilement discernable du genre Beggiatoa.

Les bactéries qui viennent d'être définies, et dont plusieurs ont déjà été décrites, ont généralement un habitat aquatique, et peuvent être trouvées notamment dans les sources d'eau thermale.

Parmi les bactéries utilisables, on peut citer par exemple :
- Vitreoscilla beggiatoïdes (ATCC 43181)
- Beggiatoa alba (ATCC 33555).

On sait que la culture des bactéries filamenteuses non photosynthétiques et non fructifiantes est relativement difficile, de même que l'obtention de cultures pures. La plupart des auteurs préconisent l'utilisation de milieux mal définis, y compris des macérations diverses utilisant l'eau de ville. La source de carbone recommandée est un acétate.

On a maintenant découvert qu'il est possible d'adapter ces bactéries, par contre-sélection, à l'utilisation d'un ose, au lieu d'acétate, comme source de carbone.

On a découvert en outre qu'il est possible de cultiver ces bactéries sur un milieu de culture parfaitement défini. On peut en particulier effectuer une culture dans le milieu suivant :

**TABLEAU 1**

| **COMPOSITION** | **CONCENTRATION** |
|---|---|
| Extrait autolytique de levures | 0,5 à 5 g/l |
| Peptone | 0,5 à 5 g/l |
| Glucose anhydre | 0,5 à 7 g/l |
| Micro-éléments de Heller | 0,5 à 5 ml/l |
| CaCl₂, 10 H₂O | 0,010 à 0,200 g/l |

On complète à 1 000 ml par de l'eau distillée. Parmi les peptones utilisables, on peut citer par exemple la peptone papaïnique de soja.

Ce milieu particulier se distingue des milieux généralement utilisés par l'absence de catalase et de sulfure.

Les micro-éléments de Heller, dont la composition est donnée dans la partie expérimentale ci-après, ont été décrits par Heller, Ann Sci. Nat. Biol. Veg. 14:1-223 (1953). Il s'agit de mélanges de divers éléments minéraux qui ont été recommandés par Heller, non pas pour la culture des bactéries, mais pour la nutrition des tissus végétaux cultivés in vitro. Il convient de noter ici que l'on n'a pas cherché à déterminer si les micro-éléments de Heller sont tous indispensables ou utiles dans la culture des bactéries filamenteuses non photosynthétiques et non fructifiantes. Il a cependant été trouvé que les micro-éléments de Heller, utilisés ensemble, par exemple en combinaison avec les amies constituants mentionnés dans le tableau 1 ci-dessus. permettent effectivement la culture des bactéries considérées.

La culture peut être effectuée à la température appropriée convenant pour l'espèce bactérienne cultivée. Généralement cette température est comprise entre 18 et 40°C suivant les souches. Le pH du milieu de culture est de préférence compris entre 5,5 et 8..

Il a été ainsi possible d'obtenir, à partir de prélèvements d'eaux thermales, des cultures axéniques, en dépit des difficultés connues d'obtention de cultures pures de bactéries filamenteuses non photosynthétiques et non fructifiantes.

On peut cultiver ces bactéries par un procédé de culture en milieu aqueux principalement caractérisé par le fait que l'on effectue la culture en utilisant un ose, par exemple le glucose, comme source de carbone principale. On cultive donc ainsi des souches ayant été adaptées et sélectionnées, comme indiqué ci-dessus, pour l'utilisation d'un ose, au lieu d'acétate, comme source principale de carbone. On peut donc effectuer la culture en l'absence d'acétate. Selon d'autres modes de réalisation, on peut en outre opérer en l'absence de H₂S (ou de sulfures) et/ou en l'absence de catalase, alors que ces ingrédients étaient généralement considérés comme indispensables jusqu'à présent. On peut également opérer en présence de micro-éléments de Heller, par exemple dans les proportions indiquées au Tableau 1. On peut utiliser notamment le milieu de culture défini au Tableau 1.

Il est donc possible d'obtenir un médicament tel que défini ci-dessus par un procédé caractérisé par le fait que l'on cultive lesdites bactéries dans un milieu de culture approprié, puis que l'on sélectionne et cultive les souches capables de se multiplier en utilisant un ose comme source principale de carbone, que l'on sépare la biomasse ou les enveloppes bactériennes, et que l'on extrait éventuellement des fractions desdites enveloppes, notamment des fractions lipopolysaccharidiques, selon les méthodes connues.

Lorsque l'on utilise des souches non encore adaptées, par exemple des souches ou des mélanges de souches provenant de prélèvement d'eaux thermales ou d'eau de mer, on les cultive d'abord dans un milieu de culture classique, contenant de l'acétate (par exemple acétate de sodium) comme seule source de carbone. On sélectionne ensuite les souches capables de se multiplier en utilisant un ose, par exemple le glucose, comme seule source de carbone, en particulier par culture dans le milieu défini qui a été indiqué précédemment. Pour la sélection et l'obtention de cultures pures, on utilise, de façon connue, les méthodes d'ensemencement de milieux de culture solides (gel d'agar) l'aide de dilutions de milieux de culture liquide, afin d'isoler des colonies issues d'une même cellule bactérienne.

Après culture des bactéries, on peut isoler la biomasse par diverses méthodes connues, par exemple par filtration, par coagulation avec un alcool (éthanol, isopropanol, isobutanol), par séchage sur cylindre à précouche (amidon, diatomées...) raclée, ou par lyophilisation. Une concentration préalable, par exemple à 80°C sous pression réduite, améliore cette séparation.

On peut procéder à une opération de rupture des enveloppes, par exemple par l'action des ultrasons. On peut en outre préparer des extraits ayant une activité immunostimulante, notamment à l'aide d'un alcool tel que l'éthanol ou le propanol.

On peut également préparer des extraits lipopolysaccharidiques selon les méthodes connues ; voir par exemple Noris et Ribbons, Methods in Microbiology, Vol. 5B, Academic Press (1971). La méthode généralement utilisée est la méthode bien connue dite de Westphal (ou une méthode apparentée), qui consiste à faire l'extraction avec des mélanges phénol-eau à 65°C. On soumet ensuite l'extrait à une dialyse pour éliminer le phénol.

L'invention concerne aussi un médicament contenant des enveloppes ou des fractions d'enveloppes de bactéries du genre Vitreoscilla.

L'invention a également pour objet une composition pharmaceutique caractérisée par le fait qu'elle contient comme principe actif un tel médicament.

Une composition contenant le médicament obtenu selon l'invention contient le principe actif à une concentration suffisante pour permettre l'administration de doses efficaces avec un volume acceptable de composition. Généralement, la composition contient de 0,01 à 1 % en poids de principe actif (exprimé par convention en poids de lipopolysaccharide) par rapport au poids total de la composition.

Une telle composition contient, outre le principe actif, un excipient approprié permettant son administration par voie entérale, parentérale ou locale. Elle peut être préparée selon les méthodes galéniques usuelles.

Elle peut se présenter notamment sous la forme d'une solution ou suspension dans un véhicule pharmaceutique liquide convenable, y compris un véhicule et un conditionnement permettant l'administration en aérosol. La composition liquide peut être soumise à une lyophilisation, et reconstituée au moment de l'emploi. La composition peut être conditionnée sous la forme de suspension buvable ou injectable, ou pour l'application locale, ou encore sous la forme d'une composition conditionnée pour l'application par voie nasale ou conjonctivale.

La composition peut être encore présentée par exemple sous la forme de gélules, de comprimés, de poudres, de suppositoires, de pâtes gingivales ou de crèmes.

Elle est utilisable notamment comme médicament immunostimulant, chez l'homme ou chez l'animal.

Elle peut être administrée par exemple par voie parentérale (intra-péritonéale, sous-cutanée, intra-musculaire, intra-veineuse, percutanée) par voie orale, par voie nasale, par voie conjonctivale, par voie rectale ou par voie per-linguale.

Elle peut être aussi utilisée on application locale, par exemple à l'aide de comprimés à délitement buccal, notamment dans l'immunothérapie non spécifique des maladies de la cavité buccale.

La posologie usuelle, exprimée par convention en poids de lipopolysaccharide, peut aller par exemple de 1µg à 100 µg/kg de poids corporel et par jour, en une ou plusieurs administrations, selon l'affection traitée.

Le médicament obtenu selon l'invention peut être administré à titre prophylactique, dans les différents cas ci-dessus et en particulier pour la prévention des infections récidivantes de la sphère otorhinolaryngologique (ORL), et pour la prévention des risques infectieux chez les malades chroniques.

Les formes galéniques permettant l'application sur la peau, par exemple les crèmes, peuvent être utilisées dans le traitement des brûlures et plaies superficielles. Elles présentent en outre des propriétés cicatrisantes.

Le médicament obtenu selon l'invention est administré notamment à titre de traitement immunostimulant, dans le domaine ORL ou bronchopulmonaire ou dans le domaine dermatologique, dans le cas d'infections bactériennes, fongiques ou virales. Ainsi, lorsque le médicament est utilisé dans le traitement des infections ORL ou bronchopulmonaires (rhinopharyngites, laryngites, sinusites, angines, otites, bronchites...), il peut être administré par voie orale sous forme de comprimés contenant par exemple de 0,05 à 2 mg de principe actif, ou sous forme de composition injectable par exemple sous forme d'ampoule stérile contenant de 0,01 mg à 0,05 mg de principe actif ou encore sous forme de composition aérosol contenant par exemple de 1 à 5 mg de principe actif pour 10 ml (et ceci pour 200 doses). Lorsque le médicament est utilisé dans le traitement des affections dermatologiques comme les brûlures, les plaies, les ulcères ou les escarres, il est généralement utilisé sous forme de crème ou de gel contenant de 0,01 à 1 % de principe actif.

Les exemples suivants illustrent l'invention sans toutefois la limiter.

### EXEMPLE 1 :Sélection/adaptation d'une bactérie filamenteuse non photosynthétique et non fructifiante : activité pharmacologique

A la suite d'un prélèvement d'eau thermale de la source de Saint-Thomas, Pyrénées Orientales (France), différentes cultures microbiennes ont été effectuées, en utilisant l'acétate de sodium comme source de carbone.

Ces premières cultures ont été effectuées dans les conditions habituelles de recherche de microorganismes dans l'eau, la température de culture étant de 26°C.

A partir de colonies bactériennes isolées par épuisement sur milieu gélosé, on a procédé à une sélection des bactéries en cultures pures capables de s'adapter à la multiplication dans un milieu contenant du glucose comme source principale de carbone.

On a utilisé le milieu de culture suivant (Tableau 2) :

**TABLEAU 2**

| **COMPOSITION** | **CONCENTRATION** |
|---|---|
| Extrait autolytique de levure Biokar (réf. 112002) | 2 g/l |
| Peptone papaïnique de soja (origine PPS-USP Biokar réf.11601) | 2 g/l |
| Micro-éléments de Heller | 2 ml/l |
| Glucose anhydre | 2 g/l |
| CaCl₂, 10 H₂O | 0,066 g/l |
| Eau distillée | 100 ml |

Le pH est ajusté à 7,15 par addition de soude ou potasse 1N avant stérilisation à 121°C pendant 20 min. La composition des micro-éléments de Heller, pour 11 d'eau distillée, est la suivante:

| | |
|---|---|
| - ZnSO₄,7 H₂O | 1g |
| - MnSO₄,H₂O | 0,076 g |
| - CuSO₄,5H₂O | 0,003 g |
| - KI | 0,010 g |
| - H₃BO₃ | 1 g |
| - AlCl₃,6H₂O | 0,050 g |
| - NiCl₂,6H₂O | 0,030 g |

On a pu ainsi sélectionner diverses souches de bactéries filamenteuses non photosynthétiques et non fructifiantes. On décrit ci-après les caractéristiques d'une bactérie filamenteuse qui a ainsi été isolée :
- Filaments (trichomes) de diamètre 2-3 µm contenant des cellules séparées par des septa,
- production d'hormogonies,
- couleur blanc beige en culture,
- bio-masse isolée de couleur rosée,
- aérobie,
- Gram négative,
- n'exigeant pas de catalase,
- inclusions de poly-β-hydroxy butyrate

Ces caractéristiques ne permettent pas, dans l'état actuel des techniques et de la taxonomie bactérienne, de classer cette souche qui se situe à l'interface des deux genres Beggiatoa et Vitreoscilla. On sait que ces genres sont très similaires.

Cette souche peut être conservée par congélation directe de la culture en phase exponentielle de croissance, à - 80°C. La souche peut être décongelée par passage dans un bain-marie à 37°C.

Après plusieurs repiquages en milieu solide, la souche adaptée au milieu indiqué ci-dessus a été stabilisée.

La culture en milieu liquide glucosé permet d'obtenir des filaments parfaitement distincts alors que la culture en milieu acétate tend à donner des agrégats.

L'optimum de température de culture est entre 26 et 30°C.

L'entretien s'effectue par repiquage à 48 h sur le même milieu liquide, en utilisant 1% du volume total comme innoculum.

### Culture en fermenteur

On effectue cette culture dans un fermenteur de 600 l agité (200 tours/min) et aéré.

Le milieu de culture est celui indiqué ci-dessus, avec addition de 0,2 g/l d'un antimousse de type polyméthylsiloxane (Silbione 97350 RP). La température est régulée entre 26 et 30°C, l'optimum se situant à 29°C.

Un cycle complet de croissance s'effectue en 48h environ. Le rendement atteint est de l'ordre de 1,4 g de poids sec par litre.

L'aération est régulée par un débitmètre musique pour avoir au minimum 20 % d'oxygène dissous.

Il n'y a pratiquement plus de glucose résiduel en fin de croissance.

La biomasse est récoltée par centrifugation.

On opère dans une centrifugeuse de type industriel refroidie à 20°C et capable d'imprimer une accélération supérieure à 5000 x g. La biomasse ainsi obtenue peut être chauffée à 121°C pendant 20 min. Ce traitement casse l'enveloppe cellulaire et libère les lipopolysaccharides. On obtient ainsi un extrait brut directement utilisable que l'on peut, le cas échéant, purifier par la méthode de Westphal précédemment citée.

### Mise en évidence de l'activité immunorégulatrice sur macrophages humains en culture :

Le principe utilisé est le suivant : lorsqu'une substance immunomodulatrice est introduite dans une culture de macrophages humains, on peut observer une activation du système par la mesure du Ca⁺⁺ libre cytosolique, dont l'élévation correspond à une activation des macrophages. Le taux de calcium cytosolique est mesuré par fluorescence à l'aide de la sonde INDO-1 sous forme d'ester. La sonde pénètre dans les cellules ou elle est transformée sous l'action d'esterases en une forme liant le calcium, la formation d'un complexe avec les ions calcium s'accompagnant d'un déplacement du spectre d'émission.

On utilise une lignée leucémique myélomonocytaire humaine THP1 fournie par le Dr. Matsushima, National Cancer Institute, Fredericks, MD, USA) produite dans du milieu RPMI 1640 (GIBCO BRL FRANCE) additionné de 5 % (V/V) de sérum de veau foetal décomplémenté (SIGMA, FRANCE), de glutamine-2mM, de pyruvate de sodium 1mM, de pénicilline 50U/ml et de streptomycine 50U/ml.

Le principe actif étudié, constitué par l'extrait obtenu ci-dessus, est introduit à diverses concentrations et incubé 45 minutes à l'obscurité. Les macrophages (5.10⁶ cellules/ml) sont soumis à une incubation de 45min à 37°C à l'obscurité en présence de 4mM de la sonde INDO-1 AM (sous forme d'ester), commercialisée par France Biochem, dans un milieu composé de 140mM NaCl, 5mM KCl, 1mM CaCl₂, 1mM MgCl₂, 10mM glucose, 20mM HEPES, de sérum albumine bovine 0,1 %, pH 7,4. Les cellules sont ensuite lavées et remises en suspension à une concentration de 10⁶ cellules/ml dans le même milieu.

La fluorescence est mesurée au moyen d'un spectrofluorimètre PERKIN-ELMER LS.5B à une longueur d'onde d'émission de 410nm pour une longueur d'onde d'excitation de 331nm.

On constate que le principe actif étudié augmente la concentration de Ca⁺⁺ cytosolique, cette augmentation croissant avec la concentration du principe actif qui varie de 33 à 267µg/ml.

On peut également déterminer l'activation des macrophages par la mesure de la mobilisation des stocks intracellulaire de calcium. Cette estimation se fait après addition de la sonde INDO-1. Le calcium extracellulaire est ensuite chélaté par l'EGTA (1mM). On ajoute ensuite le principe actif étudié, à diverses concentrations. Après stabilisation du signal la concentration do calcium extra-cellulaire est restaurée par l'ajout de CaCl₂ (2mM) dans le milieu d'incubation.

On constate que le principe actif étudié, à des concentration de 33 à 267 µg/ml permet la libération des stocks intracellulaires de Ca⁺⁺.

### Mesure de la production d'interleukine 1 β

On sait que l'on peut provoquer la sécrétion d'IL1β par des lignées monocytaires à l'aide de stimuli variés, notamment les stimuli chimiques à l'aide d'esters de phorbol (PMA)

Les cellules THP1 sont incubées en l'absence ou en présence de 1 ou 2 ng/ml de PMA et de concentrations variables du principe actif étudié (variant de 10 à 100 µg/ml), pendant 24H à 37°C. La sécrétion d'IL1β est mesurée dans le surnageant de culture à l'aide d'un essai ELISA, en utilisant des plaques de polypropylène (immulon 2 Dynatech). Des immunoglobulines (IgG) de mouton anti-IL1β sont immobilisées sur les parois des puits. La révélation est effectuée à l'aide de fragments Fab' d'anticorps anti-interleukine 1β, couplés à la peroxydase. La lecture est effectuée par mesure de la densité optique à 492nM, en présence d'orthophénylènediamine, par comparaison avec une courbe standard obtenue à l'aide d'IL1β recombinante.

Les résultats sont les suivants (Tableau 3) :

**TABLEAU 3**

| Concentration Principe Actif | | | | |
|---|---|---|---|---|
| | 10 µg/ml | 100 µg/ml | 10 µg/ml | 100 µg/ml |
| Production IL1 ng/ml | 0,2 | 1,0 | 15 | 100 |
| Concentration PMA ng/ml | - | - | 1 | 2 |

En conclusion, le principe actif étudié stimule les flux de Ca⁺⁺ chez les macrophages. L'effet est rapide (moins de 2 secondes) et est dépendant de la dose. Il concerne à la fois l'influx de Ca⁺⁺ et la mobilisation des réserves intracellulaires. De plus, le principe actif étudié stimule la production d'IL1β après 24H d'incubation, ce qui montre l'absence de toxicité pour les macrophages.

### EXEMPLE 2 : Composition dermique sous forme de crème

On a préparé une crème ayant la composition suivante :

| | |
|---|---|
| - Extrait bactérien obtenu selon le procédé de préparation de l'exemple 1 | 0,3 g |
| - Huile de vaseline | 20 g |
| - Acide stéarique | 3 g |
| - Alcool cétylique | 3 g |
| - Stéarate de polyéthylèneglycol à 100 OE | 5 g |
| - Propylèneglycol | 3 g |
| - Conservateur | 0,3 g |
| - Eau purifiée qsp | 100 g |

Cette crème est appliquée à raison de trois applications par jour pendant 10 à 15 jours sur des brûlures ou des plaies, en vue d'accélérer la cicatrisation.

### EXEMPLE 3 : Solution injectable

On a préparé une solution injectable sous forme de dose unitaire ayant la composition suivante :

| | |
|---|---|
| - Extrait bactérien obtenu selon le procédé de préparation de l'exemple 1 | 0,03 g |
| - Chlorure de sodium à 0,8 % dans l'eau | 1 ml |

Cette solution injectable est utilisée dans le traitement des affections ORL ou bronchopulmonaires chroniques. La posologie est de 1 injection sous-cutanée profonde en commençant par une demi-dose puis une dose tous les 15 jours pendant 2 mois.

## Revendications

1. Utilisation d'enveloppes de bactéries, ou de fractions desdites enveloppes, comme ingrédient actif dans la préparation d'un médicament destiné à stimuler l'immunité non spécifique, caractérisée par le fait que lesdites bactéries sont choisies parmi les bactéries de l'ordre des Beggiatoales et les bactéries du genre Vitreoscilla.

2. Utilisation selon la revendication 1, caractérisée par le fait que ledit médicament se présente sous forme de solution ou de suspension dans un véhicule approprié, de gélule, de comprimé, de comprimé à délitement buccal, de poudre, de suppositoire, ou de pâte gingivale.

3. Utilisation selon la revendication 1, caractérisée par le fait que ledit médicament se présente sous une forme appropriée pour une administration par voie orale, par voie rectale, par voie perlinguale, ou par injection.

4. Utilisation selon la revendication 1, caractérisée par le fait que ledit médicament se présente sous une forme appropriée pour une application locale.

5. Utilisation selon la revendication 4, caractérisée par le fait que ledit médicament se présente sous une forme appropriée pour une administration par voie nasale, buccale, ou conjonctivale.

6. Utilisation, selon la revendication 1, d'enveloppes de bactéries, ou de fractions desdites enveloppes, comme ingrédient actif dans la préparation d'un médicament pour la prévention et le traitement d'infections de la sphère otorhinolaryngologique ou d'infections broncho-pulmonaires.

7. Utilisation selon la revendication 6, caractérisée par le fait que ledit médicament se présente sous la forme d'une composition pour aérosol.

8. Utilisation selon la revendication 6, caractérisée par le fait que ledit médicament se présente sous forme de composition injectable.

9. Utilisation selon la revendication 6, caractérisée par le fait que ledit médicament se présente sous forme de comprimé.

10. Médicament contenant des enveloppes de bactéries ou des fractions de ces enveloppes, caractérisé par le fait que lesdites bactéries sont choisies parmi les bactéries du genre Vitreoscilla.

11. Médicament selon la revendication 10, caractérisé par le fait qu'il se présente sous la forme d'un extrait alcoolique de la biomasse obtenue après culture desdites bactéries.

12. Composition pharmaceutique caractérisée par le fait qu'elle contient comme principe actif, dans un véhicule approprié, au moins un médicament tel que défini dans l'une quelconque des revendications 10 et 11.

13. Composition selon la revendication 12, caractérisée par le fait qu'elle se présente sous la forme d'une solution ou suspension dans un véhicule pharmaceutique liquide, ou sous la forme de gélule, de comprimé, de poudre, de suppositoire, de pâte gingivale ou de crème.

14. Composition selon l'une quelconque des revendications 12 et 13, caractérisée par le fait qu'elle contient un excipient approprié permettant son administration par voie orale, par voie rectale, par voie perlinguale, ou par injection.

15. Composition selon l'une quelconque de revendications 12 et 13, caractérisée par le fait qu'elle se présente sous une forme appropriée pour une application locale.

16. Composition selon la revendication 15, caractérisée par le fait qu'elle se présente sous une forme appropriée pour une administration par voie cutanée, nasale, buccale, ou conjonctivale, ou pour une application sur la peau.

17. Composition selon l'une quelconque des revendications 15 et 16, caractérisée par le fait qu'elle contient un excipient approprié permettant son administration sous forme d'aérosol.

18. Composition selon l'une quelconque des revendications 12 à 17, caractérisée par le fait qu'elle contient de 0,01 à 1 % en poids dudit principe actif.

## Claims

1. Use of envelopes of bacteria, or of fractions of the said envelopes, as active ingredient in the preparation of a medicament intended for stimulating nonspecific immunity, characterized in that the said bacteria are chosen from bacteria of the order Beggiatoales and bacteria of the genus Vitreoscilla.

2. Use according to Claim 1, characterized in that the said medicament is provided in the form of a solution or a suspension in an appropriate vehicle, of a hard gelatine capsule, a tablet, a tablet that disintegrates in the mouth, a powder, a suppository or a gingival paste.

3. Use according to Claim 1, characterized in that the said medicament is provided in a form appropriate for administration by the oral route, by the rectal route, by the perlingual route or by injection.

4. Use according to Claim 1, characterized in that the said medicament is provided in a form appropriate for local application.

5. Use according to Claim 4, characterized in that the said medicament is provided in a form appropriate for administration by the nasal, buccal or conjunctival route.

6. Use according to Claim 1, of envelopes of bacteria, or of fractions of the said envelopes, as active ingredient in the preparation of a medicament for the prevention and treatment of infections of the otorhinolaryngological sphere or of bronchopulmonary infections.

7. Use according to Claim 6, characterized in that the said medicament is provided in the form of a composition for aerosol.

8. Use according to Claim 6, characterized in that the said medicament is provided in the form of an injectable composition.

9. Use according to Claim 6, characterized in that the said medicament is provided in the form of a tablet.

10. Medicament containing envelopes of bacteria or of fractions of these envelopes, characterized in that the said bacteria are chosen from bacteria of the genus Vitreoscilla.

11. Medicament according to Claim 10, characterized in that it is provided in the form of an alcoholic extract of the biomass obtained after culturing the said bacteria.

12. Pharmaceutical composition, characterized in that it contains, as active principle, in an appropriate vehicle, at least one medicament as defined in either of Claims 10 and 11.

13. Composition according to Claim 12, characterized in that it is provided in the form of a solution or suspension in a liquid pharmaceutical vehicle, or in the form of a hard gelatine capsule, a tablet, a powder, a suppository, a gingival paste or a cream.

14. Composition according to either of Claims 12 and 13, characterized in that it contains an appropriate excipient allowing its administration by the oral route, by the rectal route, by the perlingual route or by injection.

15. Composition according to either of Claims 12 and 13, characterized in that it is provided in a form appropriate for local application.

16. Composition according to Claim 15, characterized in that it is provided in a form appropriate for administration by the cutaneous, nasal, buccal or conjunctival route, or for application to the skin.

17. Composition according to either of Claims 15 and 16, characterized in that it contains an appropriate excipient allowing its administration in aerosol form.

18. Composition according to any one of Claims 12 to 17, characterized in that it contains from 0.01 to 1% by weight of the said active principle.

## Patentansprüche

1. Verwendung von Bakterienhüllen oder von Fraktionen dieser Hüllen als Wirkstoff in der Herstellung eines Medikaments, das für die Anregung der nichtspezifischen Immunität bestimmt ist, dadurch gekennzeichnet, dass die Bakterien ausgewählt werden unter den Bakterien der Ordnung Beggiatoales und den Bakterien der Gattung Vitreoscilla.

2. Verwendung nach Anspruch 1, dadurch gekennzeichnet, dass das Medikament in Form einer Lösung oder einer Suspension in einem geeigneten Träger, einer Gelatinekapsel, einer Tablette, einer Tablette für buccalen Zerfall, eines Pulvers, eines Zäpfchens oder einer Zahnfleischpaste vorliegt.

3. Verwendung nach Anspruch 1, dadurch gekennzeichnet, dass das Medikament in einer für eine orale, rektale, perlinguale Verabreichung oder eine Verabreichung durch Injektion geeigneten Form vorliegt.

4. Verwendung nach Anspruch 1, dadurch gekennzeichnet, dass das Medikament in einer für eine lokale Anwendung geeigneten Form vorliegt.

5. Verwendung nach Anspruch 4, dadurch gekennzeichnet, dass das Medikament in einer für die nasale, buccale oder konjunktivale Verabreichung geeigneten Form vorliegt.

6. Verwendung von Bakterienhüllen oder von Fraktionen dieser Hüllen nach Anspruch 1 als Wirkstoff in der Herstellung eines Medikaments für die Prävention und die Behandlung von Infektionen im Hals-Nasen-Ohren-Bereich oder von bronchopulmonaren Infektionen.

7. Verwendung nach Anspruch 6, dadurch gekennzeichnet, dass das Medikament in Form einer Zubereitung für ein Aerosol vorliegt.

8. Verwendung nach Anspruch 6, dadurch gekennzeichnet, dass das Medikament in Form einer injizierbaren Zubereitung vorliegt.

9. Verwendung nach Anspruch 6, dadurch gekennzeichnet, dass das Medikament in Form einer Tablette vorliegt.

10. Medikament, enthaltend Bakterienhüllen oder Fraktionen dieser Hüllen, dadurch gekennzeichnet, dass die Bakterien ausgewählt werden unter den Bakterien der Gattung Vitreoscilla.

11. Medikament nach Anspruch 10, dadurch gekennzeichnet, dass es in Form eines alkoholischen Extrakts der Biomasse vorliegt, erhalten nach der Kultur der Bakterien.

12. Pharmazeutische Zubereitung, dadurch gekennzeichnet, dass diese in einem geeigneten Träger mindestens ein Medikament nach einem der Ansprüche 10 und 11 als Wirkstoff enthält.

13. Zubereitung nach Anspruch 12, dadurch gekennzeichnet, dass diese in Form einer Lösung oder einer Suspension in einem flüssigen pharmazeutischen Träger oder in Form einer Gelatinekapsel, einer Tablette, eines Pulvers, eines Zäpfchens, einer Zahnfleischpaste oder einer Creme vorliegt.

14. Zubereitung nach einem der Ansprüche 12 und 13, dadurch gekennzeichnet, dass diese einen geeigneten Arzneimittelträger (Excipienz) enthält, der die Verabreichung auf oralem, rektalem, perlingualem Wege oder durch Injektion gestattet.

15. Zubereitung nach einem der Ansprüche 12 und 13, dadurch gekennzeichnet, dass diese in einer für eine lokale Anwendung geeigneten Form vorliegt.

16. Zubereitung nach Anspruch 15, dadurch gekennzeichnet, dass diese in einer für eine perkutane, nasale, buccale oder konjunktivale Verabreichung oder für eine Anwendung auf der Haut geeigneten Form vorliegt.

17. Zubereitung nach einem der Ansprüche 15 und 16, dadurch gekennzeichnet, dass diese einen geeigneten Arzneimittelträger (Excipienz) enthält, der die Verabreichung in Form eines Aerosols gestattet.

18. Zubereitung nach einem der Ansprüche 12 bis 17, dadurch gekennzeichnet, dass diese 0,01 bis 1 Gew.-% des Wirkstoffes enthält.
